# EUROPÄISCHE PATENTSCHRIFT

(11) **EP 0 915 085 B1**
(45) Veröffentlichungstag und Bekanntmachung des Hinweises auf die Patenterteilung: **23.04.2003**
(21) Anmeldenummer: 98119421.0
(22) Anmeldetag: 14.10.1998
(51) Int. Cl.: C07C 277/08, C07C 279/14, C07C 273/18, C07C 275/70

(54) **Verfahren zur Herstellung von Kreatin**
Process for the preparation of creatine
Procédé pour la préparation de la créatine

(30) Priorität: 04.11.1997 DE 19748694
(43) Veröffentlichungstag der Anmeldung: 12.05.1999
(73) Patentinhaber: BASF AKTIENGESELLSCHAFT, 67056 Ludwigshafen (DE)
(72) Erfinder: Greindl, Thomas Dr., 67098 Bad Dürkheim (DE); Scherr, Günter Dr., 67065 Ludwigshafen (DE); Schneider, Rolf Dr., 68163 Mannheim (DE); Mundinger, Klaus Dr., 67117 Limburgerhof (DE)

(56) Entgegenhaltungen:
- GB-A- 1 294 443
- US-A- 3 551 489
- PATENT ABSTRACTS OF JAPAN vol. 4, no. 30 (C-002), 15. März 1980 (1980-03-15) & JP 55 004349 A (IWAI TOMIRO) & JP Application 19780077364
- DATABASE CHEMABS [Online] CHEMICAL ABSTRACTS SERVICE, COLUMBUS, OHIO, US STN, CAPLUS, accession no. 1983:521849, XP002117476 & RO 75 975 B (UNIVERSITATEA "BABES-BOLYAI")
- PATENT ABSTRACTS OF JAPAN vol. 5, no. 24 (C-043), 13. Februar 1981 (1981-02-13) & JP 55 151548 A (PAAMAKEMU ASIA KK)
- S. WEISS ET AL: CHEM.-ZTG., Bd. 98, Nr. 12, 1974, Seiten 617-618, XP002117414

## Beschreibung

Die vorliegende Erfindung betrifft ein Verfahren zur Herstellung von Kreatin durch Reaktion von Calciumcyanamid mit Alkoholen zu Isoharnstoffderivaten und deren Umsetzung mit Sarkosin.

Substituierte Guanidiniumverbindungen sind in der Natur weit verbreitet. Wichtige Vertreter dieser Substanzklasse sind beispielsweise Aminosäuren, wie Arginin und Kreatin. Darüberhinaus sind substituierte Guanidinverbindungen als sterisch gehinderte Basen, als Biozide sowie als Komplexliganden bekannt. Die Mehrzahl der Verbindungen dieses Typs sind aufgrund der hohen Herstellkosten in ihrer technischen Anwendbarkeit jedoch stark eingeschränkt.

Ein Beispiel für ein biologisch aktives Guanidinderivat ist Kreatin, das als "Energieträger der Zelle" zur Nahrungsergänzung im Food und Pharmabereich eingesetzt wird.

Die Herstellung von Kreatin wird z.B. in EP-A-0 754 679 und der dort zitierten weiterführenden Literatur beschrieben, wobei maximale Ausbeuten von lediglich 70% erzielt werden.

Ein Nachteil der o.g. Synthesen für Guanidiniumverbindungen ist die Verwendung wäßriger Lösungen von reinem Cyanamid. Diese Lösungen sind sehr teuer und aufgrund der Instabilität des Cyanamids im allgemeinen nicht breit verfügbar.

Die Synthese von Guanidiniumsalzen aus reinen O-Alkylisoharnstoffderivaten ist beschrieben von R. B. Fearing and S. W. *Fox* in J. Am. Chem. Soc. 76 (1954) 4382-4385.

Die Umsetzung von Sarkosin mit O-Methylisoharnstoff-Hydrochlorid, beschrieben von E. Schütte in Hoppe-Seylers Z. Physiol. Chemie 279 (1943) 52-59, liefert Kreatin in nur 21% Ausbeute.

JP 55004349 beschreibt die Umsetzung von einer Lösung von Na-Sarkosinat mit O-Methylisoharnstoff-Methylsulfat bei pH 11 zu Kreatin.

Gemeinsames Merkmal der o.g. Guanidiniumsynthesen ist die Verwendung von reinen Einsatzstoffen.

Die Herstellung von O-Alkylisoharnstoffen ist durch säurekatalysierte Umsetzung von wasserfreiem Cyanamid mit Alkoholen bereits beschrieben (H. Krommer, Chem. Ztg. 98 (1974) 617-618; J. Stieglitz, R. H. McKee, Chem. Ber. 33 (1900) 1517-1519).

Ein Nachteil dieser Reaktion besteht in dem Einsatz von teurem und schlecht verfügbarem, wasserfreiem Cyanamid.

Eine weitere Möglichkeit zur Herstellung von O-Alkylisoharnstoffen besteht in der Umsetzung von Harnstoff mit Dialkylsulfaten. So wird in JP 55004349 die Synthese von O-Methylisoharnstoff durch Alkylierung von Harnstoff mit Dimethylsulfat beschrieben.

Ein Nachteil bei der Alkylierung von Harnstoff ist die geringe Selektivität der Reaktion, d.h. die Bildung von Nebenprodukten wie z.B. N-Alkylderivaten sowie von mehrfach alkylierten Harnstoffverbindungen.

Es bestand daher die Aufgabe, ein kostengünstiges und einfach durchführbares Verfahren zur Herstellung von Kreatin auf Basis breit verfügbarer Einsatztstoffe bereitzustellen, das die oben genannten Nachteile nicht aufweist.

Diese Aufgabe wurde gelöst durch ein Verfahren zur Herstellung von Kreatin, dadurch gekennzeichnet, daß man
a) Calciumcyanamid in Form von technischem Kalkstickstoff mit einem Gehalt an Calciumcyanamid von 20 bis 95 Gew.-%, mit einem Alkohol der Formel R¹⁰-OH zu einem Isoharnstoffderivat der Formel II, 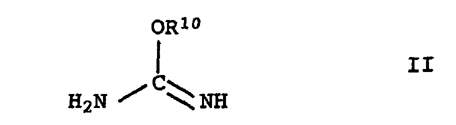 umsetzt, wobei R¹⁰ C₁-C₂₀-Alkyl bedeuten kann, und
b) den substituierten Isoharnstoff II ohne Isolierung direkt mit Sarkosin zu Kreatin umsetzt.

Das erfindungsgemäße Verfahren erfüllt insbesondere wirtschaftliche Randbedingungen, wie niedrige Einsatzstoffkosten, technisch einfache Durchführbarkeit sowie verbesserte Ausbeuten und hinreichende Reinheit des Produktes.

Das erfindungsgemäße Verfahren zeichnet sich besonders dadurch aus, daß in der ersten Stufe, d.h. für die Herstellung von O-Alkylisoharnstoffen, anstelle des teueren reinen Cyanamids, welches üblicherweise als kristallines Reinprodukt oder als eine, bei pH 3 bis 6 stabilisierte Lösung im Handel ist, sehr billiger und breit verfügbarer Kalkstickstoff eingesetzt werden kann.

Unter Kalkstickstoff versteht man Produkte, die z.B. durch Umsetzung von CaC mit N₂ bei sehr hohen Temperaturen von 800 - 1100 °C entstehen. Sie enthalten in der Regel 5 bis 98 Gew.-%, bevorzugt 20 bis 95 Gew.-%, besonders bevorzugt 30 bis 90 Gew.-% Calciumcyanamid. Der technisch erhältliche graue bis schwarze Kalkstickstoff enthält neben Calciumcyanamid zusätzlich noch Verunreinigungen, wie z.B. Kohlenstoff, Ca-Carbid, CaO sowie Spuren von Metallen üblicherweise in Anteilen <1%. Es kann selbstverständlich auch reines Ca-Cyanamid eingesetzt werden. Besonders vorteilhaft, weil wirtschaftlicher, ist jedoch der Einsatz von technischem, nicht hochreinem Kalkstickstoff. Dieser wird vorzugsweise pulverförmig, in einer Korngrößenverteilung von 1 bis 100 µm, eingesetzt. Es kann aber auch gekörntes, gestrangtes, oder anderweitig verdichtetes Material, als auch eine entsprechende Aufschlämmung in Wasser, Alkoholen oder anderen wassermischbaren Lösungsmitteln eingesetzt werden.

Die substituierten Isoharnstoffderivate der ersten Synthesestufe lassen sich durch Zugabe von Kalkstickstoff zu einem Gemisch aus 1 bis 10 Äquivalenten, bevorzugt 2 bis 8 Äquivalenten, besonders bevorzugt 3 bis 5 Äquivalenten Mineralsäure, vorzugsweise HCl, H₂SO₄ und H₃PO₄ und 1 bis 10 Äquivalenten, bevorzugt 1.5 bis 5 Äquivalenten eines Alkohols der Formel R¹⁰-OH herstellen.

Im Falle der Mineralsäuren können insbesondere auch Gemische, wie z.B. Salzsäure/Schwefelsäure oder Salzsäure/Phosphorsäure in einem Verhältnis von 20/1 bis 5/1, insbesondere 15/1 bis 8/1 verwendet werden. Diese Gemische haben den Vorteil, daß in der Reaktionsmischung vorliegende Schwermetalle gleichzeitig ausgefällt werden.

Als Alkohole der Formel R¹⁰-OH kommen solche in Frage, bei denen R¹⁰ verzweigte oder unverzweigte C₁-C₂₀-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl; 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl oder n-Eicosyl bedeuten kann.

Besonders bevorzugte Alkohole sind aliphatische Alkohole mit 1 bis 4 Kohlenstoffatome, wie z.B. Methanol, Ethanol, 1-Propanol, 2-Propanol, 1-Butanol, 2-Butanol, 3-Butanol.

Zur Entfernung gelöster Schwermetallionen kann es vorteilhaft sein, Komplexbildner wie Phosphate, Sulfate, Aminopolycarboxylate, beispielsweise EDTA oder Aminopolyphosphonate in Mengen von 0.1 bis 5 Mol.-%, bezogen auf eingesetztes Calciumcyanamid einzusetzen.

Zur Beseitigung geruchsbildender Nebenprodukte können darüber hinaus Oxidationsmittel, wie z.B. H₂O₂ zugesetzt werden.

Auf diese Weise kann die Reinheit der gebildeten Isoharnstoffderivates verbessert werden, ohne daß es zu Ausbeuteverlusten kommt.

Die Zugabe von Kalkstickstoff erfolgt in gleichmäßigen Portionen über einen Zeitraum von 0.5 bis 10 Std., bevorzugt von 1 bis 6 Std., besonders bevorzugt über einen Zeitraum von 2 bis 5 Std.

Dabei kann die Zudosierung in fester Form oder in Form einer alkoholischen Suspension erfolgen.

Die Reaktionstemperatur liegt dabei im Bereich von -20 bis 60°C, bevorzugt im Bereich von -10 bis 40°C, insbesondere im Bereich von 0 bis 25°C.

Nach der Dosierung wird in der Regel 0,5 bis 10 Std., bevorzugt 1 bis 5 Std. nachgerührt.

Eventuell in dieser Stufe ausgefallene, anorganische Nebenprodukte lassen sich bei Temperaturen von 20 bis 100°C, bevorzugt 50 bis 90°C, nach an sich bekannten Verfahren, wie z.B. Filtration oder Zentrifugation abtrennen.

Das gebildete Isoharnstoffderivat läßt sich ohne weitere Aufreinigung in einer zweiten Verfahrensstufe mit Sarkosin zu Kreatin setzen.

Sarkosin kann sowohl als freie Säure als auch insbesondere als Na- oder K-Salz in Form einer 5 bis 60 gew.-%igen, bevorzugt 35 bis 45 gew.-%igen wäßrigen Lösung verwendet werden

Der Einsatz von technischen Produkten empfiehlt sich. insbesondere dort, wo keine weiteren unerwünschten reaktiven Amine beigemischt sind und es aus wirtschaftlichen Gründen besonders vorteilhaft ist, wie beispielsweise die Aufreinigung des Amins teuer und aufwendig ist.

Die Umsetzung der substituierten Isoharnstoffderivate mit Sarkosin kann in Wasser oder einem mit Wasser mischbaren Lösungsmittel, oder einem Gemisch davon erfolgen. Üblicherweise wird dabei ein pH-Wert im Bereich des pK-Wertes des Amins verwendet, d.h. in einem pH-Bereich zwischen 6 und 14, bevorzugt zwischen 8 und 12, besonders bevorzugt im pH-Bereich von 9 bis 11.

Das Molverhältnis von substituierten Isoharnstoffderivaten zu Sarkosin liegt im Bereich von 0,9 bis 5,0, vorzugsweise im Bereich von 1,0 bis 2,0. Die Reaktionstemperaturen in der zweiten Stufe liegen im Bereich von -20 bis 100°C, bevorzugt im Bereich von 0 bis 60°C, besonders bevorzugt zwischen 10 und 40°C.

Für die Reaktionsführung in der zweiten Verfahrensstufe spielt die Reihenfolge der Zugabe der Reaktanten keine besondere Bedeutung. In der Regel gibt man den substituierten Isoharnstoff zum Sarkosin der bevorzugt in wäßriger oder alkoholischer Lösung vorliegen kann.

Die Zugabe kann sich über einen Zeitraum von 0.5 bis 10 Std., bevorzugt von 1 bis 3 Std. erstrecken.

Zur Einhaltung des pH-Wertes können je nach Ausgangs-pH-Wert der Base entweder Säuren, wie CO₂, SO₂, HCl, HNO₃, H₂SO₄, H₂SO₃, H₃PO₃, H₃PO₂ und H₃PO₄, und/oder Basen wie NaOH, KOH, LiOH, Ca(OH)₂, Ba(OH)₂, Mg(OH)₂ eingesetzt werden. Sollte das Sarkosin in basischer und nicht in neutralisierter oder teilneutralisierter Form vorliegen, so werden nur Säuren benötigt.

Als Säuren sind solche bevorzugt, die technisch leicht verfügbar sind und zusammen mit Schwermetallspuren zu schwerlöslichen Komplexen führen, wie z.B. CO₂; H₂SO₄, H₃PO₄. Es können aber auch bevorzugt Gemische dieser und anderer Säuren eingesetzt werden.

Nach Abtrennung der ggf. ausgefallenen, komplexierten Nebenprodukte, entweder durch Heißfiltration oder Zentrifugation, erfolgt die Isolierung des Kreatin in an sich bekannter Weise. So läßt sich beispielsweise durch Abkühlen der abfiltrierten Reaktionslösung auf -20 bis 60°C, insbesondere 0 bis 40°C, das Wertprodukt kristallin erhalten. Nach Filtration kann gegebenenfalls durch eine weitere Umkristallisation die Reinheit verbessert werden. Es ist aber auch möglich, daß Produkt mittels Extraktion aus dem Reaktionsgemisch zu entfernen, um es anschließend durch Destillation oder Kristallisation sauber zu isolieren.

Es ist in besonderer Weise überraschend, daß die Ausbeuten der erfindungsgemäßen Reaktion, bezogen auf den Gehalt an Cyanamid (1. Stufe) und O-Alkylisoharnstoff (2. Stufe) bei der Verwendung der technischen Ausgangsstoffe mit denen der Umsetzung von reinem Cyanamid und O-Alkylisoharnstoffen vergleichbar sind. Unter Berücksichtigung des Reinigungsschrittes für die jeweilige Herstellung von reinem Cyanamid bzw. O-Alkylisoharnstoff liegt die Ausbeute aufgrund der geringeren Zahl der Verfahrensschritte weit höher.

Zudem können noch höhere Umsätze erzielt werden, wenn man im zweiten Verfahrensschritt die billige Isoharnstoffverbindung im Überschuß zum Amin einsetzt, was bei Einsatz von reinem Cyanamid oder reinem O-Alkylisoharnstoff häufig unwirtschaftlich ist. Die erreichte Reinheit des isolierten Kreatin ist mit der aus reinem Cyanamid hergestellten Ware vergleichbar. Dies ist insbesondere auf die hohe Reinheit der erfindungsgemäß erhaltenen Isoharnstoffderivate zurückzuführen.

In dem folgenden Beispiel wird das Verfahren zur Herstellung von Kreatin näher erläutert.

### Beispiel: Herstellung von Kreatin

### Stufe 1: Synthese von O-Methylisoharnstoff Hydrochlorid

400 ml trockenes Methanol und 1 ml 85%ige Phosphorsäure wurden in einem 500 ml Rundkolben mit Rührer, Gaseinleitungsrohr, Rückflußkühler und Trockenrohr bei 0 bis 5°C mit trockenem Chlorwasserstoff bis zur Sättigung begast. Anschließend wurden unter Eiskühlung 120 g Kalkstickstoff mit einem Gehalt an CaCN₂ von 43 Gew.-% so eingetragen, daß eine Temperatur von 20°C nicht überschritten wurde. Nach der Zugabe wurde 2 Std. bei 20°C nachgerührt. Anschließend wurde das Reaktionsgemisch unter Rückfluß aufgekocht, die gebildete Suspension abfiltriert und der Filterkuchen dreimal mit je 50 ml Methanol gewaschen. Die vereinigten Mutterlaugen wurden im Vakuum bis auf 200 ml aufkonzentriert. Nach HPLC-Analyse enthielt das Konzentrat 61,2 g O-Methylisoharnstoff Hydrochlorid. entsprechend einer Ausbeute von 88%.

### Stufe 2: Umsetzung von O-Methylisoharnstoff Hydrochlorid mit Sarkosin

Zu einem Gemisch von 138 g 40,1 Gew.-%iger wäßriger Na-Sarkosinat-Lösung, 16,2 g 50 Gew.-%iger Schwefelsäure und 53 g Wasser bei pH 11 wurde die in der ersten Stufe hergestellte methanolische O-Methylisoharnstoff-Hydrochlorid-Lösung innerhalb von 2 Std. bei einer Temperatur von 20°C zudosiert. Der pH-Wert wurde während der Dosierung durch gleichzeitige Zugabe von 20 gew.-%iger NaOH bei 11 gehalten. Nach Dosierende wurde das Reaktionsgemisch noch 6 Std. bei 20°C nachgerührt und anschließend das Lösungsmittel im Vakuum bei 60°C bis auf ca. 150 ml abdestilliert. Aus dem verbleibenden Rückstand erhielt man nach Kristallisation 62,5 g Kreatin mit einer Reinheit von 88% und einem Restwassergehalt von 12%. Nach Analyse der Mutterlauge lag die Gesamtreaktionsausbeute an Kreatin bei 87%.

## Patentansprüche

1. Verfahren zur Herstellung von Kreatin, **dadurch gekennzeich-net, daß** man
a) Calciumcyanamid in Form von technischem Kalkstickstoff mit einem Gehalt an Calciumcyanamid von 20 bis 95 Gew.-%, mit einem Alkohol der Formel R¹⁰-OH zu einem Isoharnstoffderivat der Formel II, 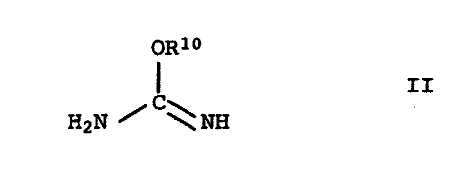 umsetzt, wobei R¹⁰ C₁-C₂₀-Alkyl bedeuten kann, und
b) den substituierten Isoharnstoff II ohne Isolierung direkt mit Sarkosin zu Kreatin umsetzt.

2. Verfahren nach Anspruch 1, **dadurch gekennzeichnet, daß** man Calciumcyanamid im Verfahrensschritt a) mit einem Alkohol der Formel R¹⁰-OH bei einer Temperatur von -20 bis 60°C in Gegenwart von Mineralsäuren umsetzt.

3. Verfahren nach den Ansprüchen 1 und 2, **dadurch gekennzeichnet, daß** der Verfahrensschritt b) bei einer Temperatur von -20 bis 100°C und einem pH-Wert von 6 bis 14 durchgeführt wird.

4. Verfahren nach den Ansprüchen 1 bis 3, **dadurch gekennzeichnet, daß** man Sarkosin als Na- oder K-Salz in Form einer 5 bis 60 Gew.-%igen wäßrigen Lösung verwendet.

## Claims

1. A process for preparing creatine,
which comprises
a) reacting calcium cyanamide in the form of technical nitrolime with a calcium cyanamide content of from 20 to 95% by weight with an alcohol of the formula R¹⁰-OH to give an isourea derivative of the formula II, 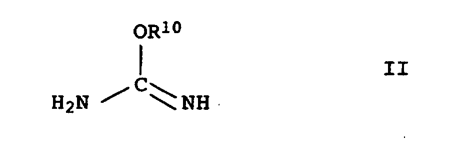 where R¹⁰ can be C₁-C₂₀-alkyl, and
b) reacting the substituted isourea II without isolation directly with sarcosine to give creatine.

2. A process as claimed in claim 1, wherein calcium cyanamide is reacted in step a) with an alcohol of the formula R¹⁰-OH at from -20 to 60°C in the presence of mineral acids.

3. A process as claimed in either of claims 1 or 2, wherein step b) is carried out at from -20 to 100°C and at a pH of from 6 to 14.

4. A process as claimed in any of claims 1 to 3, wherein sarcosine is used as Na or K salt in the form of a 5 to 60% by weight aqueous solution.

## Revendications

1. Procédé pour la préparation de créatine, **caractérisé par le fait qu'**on
a) fait réagir du cyanamide calcique sous forme de chaux azotée technique ayant une teneur en cyanamide calcique de 20 à 95% en poids, avec un alcool de formule R¹⁰-OH pour donner un dérivé d'isourée de formule II, 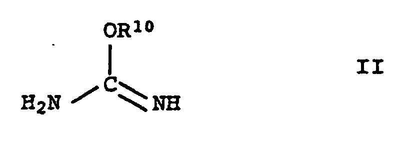 où R¹⁰ peut désigner un alkyle en C₁-C₂₀, et
b) fait réagir l'isourée II substituée sans isolement, directement avec la sarcosine pour former la créatine.

2. Procédé selon la revendication 1, **caractérisé par le fait qu'**on fait réagir le cyanamide calcique dans l'étape opératoire a) avec un alcool dé formule R¹⁰-OH à une température de -20 à 60°C en présence d'acides minéraux.

3. Procédé selon les revendications 1 et 2, **caractérisé par le fait qu'**on effectue l'étape opératoire b) à une température de -20 à 100°C et à un pH de 6 à 14.

4. Procédé selon les revendications 1 à 3, **caractérisé par le fait qu'**on utilise la sarcosine à l'état de sel de Na ou de K, sous la forme d'une solution aqueuse à 5 à 60% en poids.
